# EUROPEAN PATENT SPECIFICATION

(11) **EP 2 276 454 B1**
(45) Date of publication and mention of the grant of the patent: **24.12.2014**
(21) Application number: 09745818.6
(22) Date of filing: 15.05.2009
(51) Int. Cl.: A61K 8/35, A61Q 17/04, A61K 8/49, A61K 8/58

(54) **SUNSCREEN COMPOSITIONS**
SONNENSCHUTZZUSAMMENSETZUNGEN
COMPOSITIONS DE PROTECTION SOLAIRE

(30) Priority: 16.05.2008 EP 08009079
(43) Date of publication of application: 26.01.2011
(73) Proprietor: DSM IP Assets B.V., 6411 TE Heerlen (NL)
(72) Inventor: JANSSEN, Anne, CH-4127 Birsfelden (CH); LENZ, Dirk, CH-4059 Basel (CH); MENDROK-EDINGER, Christine, 79618 Rheinfelden-Minseln (DE)
(74) Representative: Berg, Katja
(86) International application number: PCT/EP2009/055903
(87) International publication number: WO 2009/138486

(56) References cited:
- WO-A-2007/065574
- WO-A1-2005/048960

## Description

The present invention relates to topical compositions comprising at least 3 wt.-% of polysilicones-15, at least 4.5 wt.-% of butyl methoxydibenzoylmethane and at least 3 wt.-% of phenylbenzimidazol sulfonic acid in combination with at least 10 wt.-%, preferably at least 12 wt.-% of at least one further UVB and/ or broadband-filter substance selected from the group consisting of octocrylene, ethyl hexylsalicylate, homosalate, unsymmetrical s-triazine derivatives, 2-Hydroxy-4-methoxy-benzophenon, 2,2'-Methylen-bis-(6-(2H-benzotriazol-2-yl)-4-(1,1,3,3-tetramethyl-butyl)-phenol), and titanium dioxide.

Sun care products have evolved considerably over the years. Earlier formulations were intended to protect the user from UV-B radiation (UVB) as was once thought that UV-B rays were the most important contributors to wrinkling, skin disease, and skin cancer. However, more recent studies have shown that UV-A radiation (UVA) is equally or even more important in the development of solar damage and skin diseases, such as lupus erythematosus and melanoma and non-melanoma skin cancers. Thus, today's focus is toward eliminating as much of UVA (320-400 nm) and / or UVB (280-320 nm) light as possible. Consequently, there's a constantly increasing need for sun care products exhibiting high SPF's up to 50+ and high UVA protection in a population which is exposed to an increasing amount of damaging sunlight.

The SPF (Sun Protection Factor) rating system has been developed to provide consumer guidance in selecting sun care products. SPF is measured in the laboratory with a solar simulator that induces UV erythema. However, erythema is primarily caused by UVB, making SPF testing primarily a measurement of UVB - not UVA - protection.

Colipa has developed a method for testing the UVA performance of sun care products that meet the European Commission's Recommendation of 22 September 2006 on the efficacy of sunscreen products. The UVA protection performance according to the invention is determined according to the 'Method for the in vitro determination of UVA protection provided by sunscreen products' (COLIPA Guideline 2007) which is labeled as UVAPF (UVA protection factor).

Another international rating system for UVA protection is the critical wavelength. Sun care products with a critical wavelength over 370nm are considered by the FDA to provide excellent UVA protection.

Many UV-filter substances have been developed in the past protecting against the harmful effect of UVA and / or UVB radiation and even shorter radiation (UVC). These substances are usually incorporated either alone or in combination with each other into cosmetic or pharmaceutical preparations which are widely known and used.

Due to the increasing demand for high SPF sun care products, in particular SPF 50+ sun care products with a UVA protection complying with the above mentioned standards, more UV-filter substances at elevated levels have to be incorporated into the sun care products; this, however, is not always feasible, as high UV-filter substance concentrations add considerable cost to the formulation and often lead to an unpleasant skin feel and/ or aesthetic appearance. Furthermore, high sunscreen levels can promote increased irritancy. Additionally not all UVA-filter substances are equally suited to achieve the recommended UVA protection at a reasonable concentration level. Thus, there is an ongoing need for stable sun care products exhibiting a high SPF, in particular a SPF of 50+ and a UVA protection meeting the above mentioned standards at comparatively low UV-filter concentrations. A further demand is that the sun care product, despite using butyl methoxydibenzoylmethane exhibits a sufficient photostability, shows no irritancy and exhibits a pleasing skin feel and aesthetic appearance.

WO2005/048960 A1 discloses for example light protecting compositions containing at least one polysiloxane-based UV filter with an increased ratio of the sunprotecting factor to the total UV filter amount.

Surprisingly, it has been found that it is possible to formulate stable, high SPF sun care products, in particular SPF 50 and even SPF 50+ sun care products with a UVA protection complying with the above mentioned standards at comparatively low UV-filter substance concentrations (illustrated by a ratio of the in vivo SPF/ UV-filter concentration of about 2 to 3) using a specific combination of polysilicones-15, phenylbenzimidazol sulfonic acid and butyl methoxydibenzoylmethane.

Thus, the invention relates to topical compositions comprising at least 3 wt.-% of polysilicones-15, at least 3 wt.-% of phenylbenzimidazol sulfonic acid, at least 4.5 wt.-% of butyl methoxydibenzoylmethane and at least 10 wt.-%, preferably at least 12 wt.-% of at least one further UVB and/ or broadband-filter substance in a cosmetically acceptable carrier, characterized in that the at least one further UVB and/ or broadband-filter substance is selected from the group consisting of octocylene, ethyl hexylsalicylate, homosalate, unsymmetrical s-triazine derivatives, 2-Hydroxy-4-methoxy-benzophenon, 2,2'-Methylen-bis-(6-(2H-benzotriazol-2-yl)-4-(1,1,3,3-tetramethyl-butyl)-phenol), and titanium dioxide. Preferably, at least two further UVB and/ or broadband-filter substance are used in the topical compositions according to the invention.

In another aspect, the invention relates to topical compositions comprising 3 wt.-% of polysilicones-15, 4 wt.-% of phenylbenzimidazol sulfonic acid, 5 wt.-% of butyl methoxydibenzoylmethane and at least 10 wt.-%, preferably at least 12 wt.-% of at least one, preferably at least two further UVB and/ or broadband-filter substance in a cosmetically acceptable carrier, characterized in that the at least one further UVB and/ or broadband-filter substance is selected from the group consisting of octocrylene, ethyl hexylsalicylate, homosalate, unsymmetrical s-triazine derivatives, 2-Hydroxy-4-methoxy-benzophenon, 2,2'-Methylen-bis-(6-(2H-benzotriazol-2-yl)-4-(1,1,3,3-tetramethyl-butyl)-phenol), and titanium dioxide.

The topical compositions according to the invention overcome the drawbacks of the prior art as outlined above. In particular, the compositions are easy to apply to the skin, are little, respectively not sticky and exhibit a good skin feel while complying with the recommended UV protection standards.

The at least one further UVB and/ or broadband-filter substance is selected from conventional UVB and/ or broad spectrum UV-filter substances of the group consisting of octocrylene ethyl hexylsalicylate, homosalate, unsymmetrical s-triazine derivatives. 2-Hydroxy-4-methoxy-benzophenon, 2,2'-Methylen-bis-(6-(2H-benzotriazol-2-yl)-4-(1,1,3,3-tetramethyl-butyl)-phenol), and titanium dioxide known to be added into topical compositions such as cosmetic or dermatological sun care products. Such UV-filter substances are advantageously selected from among the compounds listed below without being limited thereto:

Examples of UVB or broad spectrum UV-filter substances, i.e. substances having absorption maximums between about 290 nm and 340 nm may be organic or inorganic compounds. Organic UV-B or broad spectrum UV-filter substances are e.g. acrylates such as 2-ethylhexyl 2-cyano-3,3-diphenylacrylate (octocrylene, PARSOL^{®} 340), ethyl 2-cyano-3,3-diphenylacrylate and the like; Camphor derivatives such as 4-methyl benzylidene camphor (PARSOL^{®} 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like; Cinnamate derivatives such as ethylhexyl methoxycinnamate (PARSOL^{®} MCX), ethoxyethyl methoxycinnamate, isoamyl methoxycinnamate and the like as well as cinnamic acid derivatives bond to siloxanes; p-Aminobenzoic acid derivatives, such as p-aminobenzoic acid, 2-ethylhexyl p-dimethylaminobenzoate, N-oxypropylenated ethyl p-aminobenzoate, glyceryl p-aminobenzoate; Benzophenones such as benzophenone-3, benzophenone-4, 2,2',4,4'-tetrahydroxy-benzophenone, 2,2'-dihydroxy-4,4'-dimethoxybenzophenone and the like; Esters of benzalmalonic acid such as di-(2-ethylhexyl) 4-methoxybenzalmalonate; Drometrizole trisiloxane (Mexoryl XL); Imidazole derivatives; Salicylate derivatives such as isopropylbenzyl salicylate, benzyl salicylate, butyl salicylate, ethylhexyl salicylate (PARSOL^{®} EHS, Neo Heliopan OS), isooctyl salicylate or homomenthyl salicylate (homosalate, PARSOL^{®} HMS, Neo Heliopan HMS) and the like; Triazine derivatives such as e.g. ethylhexyl triazone (Uvinul^{®} T-150), diethylhexyl butamido triazone (Uvasorb^{®} HEB), bis-ethylhexyloxyphenol methoxyphenyl triazine (Tinosorb^{®} S); Benzotriazole derivatives such as e.g. 2,2'-methylene-bis-(6-(2H-benzotriazole-2-yl)-4-(1,1,3,3,-tetramethylbutyl)-phenol (Tinosorb^{®} M); Encapsulated UVB and/ or broadband-filter substances such as e.g. encapsulated ethylhexyl methoxycinnamate (Eusolex^{®} UV-pearls) or microcapsules loaded with UV-filters as e.g. dislosed in EP 1471995; Inorganic UVB or broad spectrum UV-filter substances useful herein include metallic oxides, in particular titanium dioxide having an average primary particle size of from about 15 nm to about 100 nm and/ or zinc oxide having an average primary particle size of from about 15 nm to about 150 nm. The particles may also be coated by other metal oxides such as e.g. aluminum or zirconium oxides or by organic coatings such as e.g. polyols, methicone, aluminum stearate, alkyl silane. Such coatings are well known in the art. Particular preferred titanium dioxide is a double coated titanium dioxide such as e.g. silica and dimethicone coated titanium dioxide having a rutile crystal structure e.g. available as PARSOL TX at DSM Nutritional Products.

Generally, the amount of each UVB and/ or broad spectrum UV-filter substances in the topical compositions according to the invention is selected in the range of about 0.1 to 15 wt.-%, preferably in the range of about 0.2 to 10 wt.-%, most preferably in the range of about 0.5 to 8 wt.-% with respect to the total weigh of the topical composition.

The total amount of UV-filter substances in the topical compositions according to the invention is preferably in the range of about 20 to 30 wt.-% with respect to the total weight of the topical composition.

Preferred UVB-filter substances according to the invention encompass octocrylene, ethyl hexylsalicylate and/ or homosalate.

Preferred broadband UV-filter substances according to the invention encompass unsymmetrical s-triazine derivatives such 2,4-Bis-{[4-(2-ethyl-hexyloxy)-2-hydroxy]-phenyl}-6-(4-methoxyphenyl)-1,3,5-triazin, certain benzophenones such as e.g. 2-Hydroxy-4-methoxy-benzophenon, 2,2'-Methylen-bis-(6-(2H-benzotriazol-2-yl)-4-(1,1,3,3-tetramethyl-butyl)-phenol), and/ or titanium dioxide.

In a particular preferred embodiment the at least one further UVB and/ or broadband-filter substance is octocrylene. Preferably, the topical compositions according to the invention comprising octocrylene further comprise at least one additional UVB and/ or broadband-filter substance which is preferably selected from titanium dioxide, ethylhexyl salicylate and/ or homosalate. Most preferably, the topical compositions according to the invention comprise 1 to 10 wt.-%, in particular 3 to 10 wt.-%, preferably 3.6 to 8 wt.-% of octocrylene and at least one further UVB and/ or broadband-filter substance, preferably selected from titanium dioxide, ethylhexyl salicylate and/ or homosalate.

In a preferred embodiment the total amount of UV-filter substances in the topical compositions according to the invention is in the range of 20 to 35 wt.-%, in particular in the range of 22 to 30 wt.-% with respect to the total weigh of the topical composition and the ratio of the in vivo SPF to the UV-filter concentration is in the range of about 2 to 3.

In another preferred embodiment, the topical compositions according to the invention exhibit an in vivo SPF of over 40, in particular of over 50. Further preferred are topical compositions according to the invention exhibiting an in vivo SPF of over 40, in particular of over 50, showing an UVA protection factor of at least 1/3 of the SPF (determined according to the 'Method for the in vitro determination of UVA protection provided by sunscreen products' (COLIPA Guideline 2007)) and having a critical wavelength over 370nm.

Surprisingly, it has furthermore been found that the SPF can be further increased by using a phosphate ester surfactant. Thus, in a particular embodiment the invention relates to topical compositions according to the invention further comprising a phosphate ester surfactant. Suitable phosphate esters surfactants have the formula I

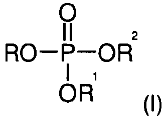

The phosphate ester surfactant has the general structure wherein R, R¹ and R² may be hydrogen, an alkyl of from 1 to about 22 carbons, preferably from about 12 to 18 carbons, or an alkoxylated alkyl of from 1 to about 22 carbons, preferably from about 12 to 18 carbons, and having 1 or more, preferably from about 2 to about 25, most preferably 2 to 12, moles ethylene oxide, with the proviso that at least one of R, R¹ and R² is an alkyl or alkoxylated alkyl as previously defined but having at least 6 alkyl carbons in said alkyl or alkoxylated alkyl group.

Monoesters in which R¹ and R² are hydrogen and R is selected from alkyls of 10 to 18 carbons and alkoxylated fatty alcohols of 10 to 18 carbons and 2 to 12 moles ethylene oxide are preferred. Among the preferred phosphate ester surfactants, mention may be made of C12-16 Pareth-6 Phosphate, C8-10 Alkyl Ethyl Phosphate, C9-15 Alkyl Phosphate, Ceteareth-2 Phosphate, Ceteareth-4 Phosphate, Ceteareth-5 Phosphate, Ceteareth-10 Phosphate, Ceteth-8 Phosphate, Ceteth-10 Phosphate, Cetyl Phosphate, C6-10 Pareth-4 Phosphate, C12-13 Pareth-10 Phosphate, C12-15 Pareth-2 Phosphate, C12-15 Pareth-3 Phosphate, C12-15 Pareth-6 Phosphate, C12-15 Pareth-8 Phosphate, C12-15 Pareth-10 Phosphate, C12-16 Pareth-6 Phosphate, DEA-Ceteareth-2 Phosphate, DEA-Cetyl Phosphate, DEA-Oleth-3 Phosphate, DEA-Oleth-5 Phosphate, DEA-Oleth-10 Phosphate, DEA-Oleth-20 Phosphate, Potassium cetyl phosphate, Deceth-9 Phosphate, Deceth-4 Phosphate and Deceth-6 Phosphate. Particular preferred phosphate ester surfactants according to the invention are cetyl phosphate, potassium cetyl phosphate and/ or DEA cetyl phosphate.

The phosphate ester surfactant is generally present in the topical compositions according to the invention in proportions ranging from 0.1 to 5 wt.-%, preferably from 0.5 to 5 wt.-%, most preferably from 2 to 3 wt.-% with respect to the total weigh of the topical composition.

In a particular preferred embodiment, the invention relates to a topical composition comprising 3 wt.-% of polysilicones-15, 4 wt.-% of phenylbenzimidazol sulfonic acid, 5 wt.-% of butyl methoxydibenzoylmethane, at least 10 wt.-%, preferably at least 12 wt.-% of at least one, preferably at least two further UVB and/ or broadband-filter substances and a phosphate ester surfactant in a cosmetically acceptable carrier. Preferably, the at least one further UVB and/ or broadband-filter substance is octocrylene used in a concentration from about 3 to 10 wt.-%, preferably from about 3.6 to 8 wt.-% and the phosphate ester surfactant is cetyl phosphate, potassium cetyl phosphate, and/ or DEA cetyl phosphate used in a concentration of about 2 to 3 wt.-% with respect to the total weigh of the topical composition and the further additional UVB and/ or broadband-filter substances are selected from titanium dioxide, ethylhexyl salicylate and/ or homosalate.

In a particular embodiment all topical compositions according to the invention further comprise a co-surfactant in an amount of 1 to 4 wt.-%, in particular about 2 wt.-% in order to further improve the stability of the formulation. Preferably, the co-surfactant is selected from Lanette O (Cetearyl Alcohol (mixture of C16 and C18 alcohol)), Lanette 16 / Lorol C 16 (Cetyl Alcohol) and/ or Estol 3650 (Glyceryl Myristate).

The term "topical composition" as used herein refers in particular to cosmetic compositions that can be topically applied to mammalian keratinous tissue such as e.g. human skin or hair, particularly human skin.

The term "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic preparations as disclosed in A. Domsch, "Cosmetic Preparations", Verlag für chemische Industrie (ed. H. Ziolkowsky), 4th edition, 1992.

The term cosmetically acceptable carrier refers to all carriers and/or excipients and/ or diluents conventionally used in topical preparations

Preferably, the topical preparations according to the present invention are in the form of a suspension or dispersion in solvents or fatty substances, or alternatively in the form of an emulsion or micro emulsion (in particular of O/W- or W/O-type), PIT-emulsion, multiple emulsion (e. g. O/W/O- or W/O/W-type), pickering emulsion, hydrogel, alcoholic gel, lipogel, one- or multiphase solution or vesicular dispersion or other usual forms, which can also be applied by pens, as masks or as sprays. If the topical preparation is or comprises an emulsion it can also contain one or more anionic, nonionic, cationic or amphoteric surfactant(s).

In a particular embodiment, the topical compositions according to the invention are O/W emulsions. Preferably, the surfactant of the O/W emulsion are selected from the group of glycerylstearate in combination with ceteareth-20 and/ or ceteareth-25, ceteareth-6 in combination with stearylalcohol, cetylstearylalcohol in combination with PEG-40-ricinusoil and sodiumcetylstearylsulfate, triceteareth-4 phosphate, glycerylstearate, sodiumcetylstearylsulfate, lecithin trilaureth-4 phosphate, laureth-4 phosphate, stearic acid, propylenglycolstearate SE, PEG-25-hydrated ricinus oil, PEG-54-hydrated ricinus oil and/ or PEG-6 caprylic acid/caprinic acid glycerides, glyceryloleate in combination with propylenglycole, PEG-9-Stearate, PEG-20 Stearate, PEG-30-Stearate, PEG-40-stearate, PEG-100-stearate, ceteth-2, ceteth-20, polysorbate-20, polysorbate-60, polysorbate-65 and/ or polysorbate-100, glycerylstearate in combination with PEG-100 stearate, glycerylmyristate, glyceryllaurate, PEG-40-Sorbitanperoleat, Laureth-4, Ceteareth-3 and/ isostearylglycerylether, cetylstearylalkohol in combination with sodium cetylstearylsulfat, laureth-23 and/ or steareth-2, glycerylstearat in combination with PEG-30 stearate, PEG-40-stearate, glycol distearate, PEG-22-dodecyl glycol Copolymer, polyglyceryl-2-PEG-4-stearat, ceteareth-12, ceteareth-20, ceteareth-30, methyl-glucosesesquistearate, steareth-10 and/ or PEG-20-stearat, steareth-2 in combination with PEG-8 distearate, steareth-21, steareth-20, isosteareth-20, PEG-45/ dodecylglycol-copolymer, methoxy-PEG-22/dodecylglycol-copolymer, PEG-40-sorbitanperoleat, PEG-40-sorbitanperisostearats, PEG-20-glycerylstearats, PEG-20-glycerylstearats, PEG-8-bee wax, polyglyceryl-2-laurate, isostearyldiglycerylsuccinat, stearamidopropyl-PG-dimoniumchloridphosphat, ceteth-20, glycerylstearate SE, triethylcitrate, PEG-20-methylglucosesesquistearat, cetylphosphate, glycerylstearatcitrate, cetearyl sulfate, sorbitansesquioleate, triceteareth-4-phosphats, trilaureth-4-phosphate, polyglycerylmethylglucosedistearate, potassium cetylphosphate, polyglyceryl-3 methylglucose distearate, isosteareth-10, polyglyceryl-2-sesquiisostearate, ceteth-10, oleth-20 and/ or isoceteth-20, glycerylstearate in combination with ceteareth-20, ceteareth-12, cetylstearylalcohol and/ or cetylpalmitate, cetylstearylalcohol in combination with PEG-20 stearate, PEG-30-stearate, PEG-40-stearate and/ or PEG-100-stearate. Particularly preferred O/W surfactants are phosphate ester surfactants such as cetyl phosphate, potassium cetyl phosphate and/ or DEA cetyl phosphate, glycerylstearate, PEG-40 stearate, PEG-100 stearate in combination with glyceryl stearate, triglycerinmethylglucosedistearate, polyglyceryl-3 methylglucose distearate, cetearylglucoside, polyethylenglycol(21)stearylether, polyethylenglycol(2)stearylether, glycerylstearatcitrate, sodium cetearyl-sulfate, setearylalkohol, stearic acid and/ or sorbitanstearate.
Particularly preferred O/W surfactants according to the invention are phosphate ester surfactant, in particular selected from cetyl phosphate, potassium cetyl phosphate and/ or DEA cetyl phosphate and/ or PEG-100 stearate in combination with glyceryl stearate. The most preferred O/W surfactants according to the invention are the phosphate ester surfactants cetyl phosphate, potassium cetyl phosphate and/ or DEA cetyl phosphate.

The oil phase of the O/W emulsions according to the invention preferably comprises oils selected from butylenglykoldicaprylat/-dicaprat, dicaprylylether, C₁₂₋₁₅-Alkylbenzoat, C₁₈₋₃₈-fatty acid triglyceride, dibutyladipate, cyclomethicone, 2-phenylethylbenzoat, isopropyl lauroyl sarkosinate, diisopropyl sebacate as well as mixtures thereof. The amount of the oil (one or several) in the O/W emulsion according to the invention is generally selected in the range of about 0,1 bis 40 wt.-%, in particular in the range of about 1,0 to 30 wt.-%, most in particular in the range of about 5% to 20 wt.-% with respect to the total weight of the topical composition.

Preferred topical compositions according to the invention are sun care preparations.

Topical compositions in accordance with the invention can be in the form of a liquid, lotion, a thickened lotion, a gel, a cream, a milk, an ointment, a paste, a powder, a make-up, or a solid tube stick and can be optionally be packaged as an aerosol and can be provided in the form of a mousse such as a aerosol mousse, a foam or a spray foam, a spray, a stick.

The topical cosmetic compositions of the invention can also contain usual cosmetic adjuvants and additives such as e.g. further UVA filter substances, preservatives, antioxidants, fatty substances, oils, water, alcohols, polyols, organic solvents, electrolytes, silicones, thickeners, film forming agents, softeners, emulsifiers, complexing agents, antifoaming agents, moisturizers, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, essential oils, skin sensates, astringents, antifoaming agents, pigments or nanopigments, cosmetically active ingredients or any other ingredients, carriers and/or excipients or diluents conventionally formulated into cosmetic compositions. Such cosmetic ingredients commonly used in the skin care industry, which are suitable for use in the compositions of the present invention are e.g. described in the CTFA Cosmetic Ingredient Handbook, Second Edition (1992) without being limited thereto.

The necessary amounts of the cosmetic and dermatological adjuvants and additives can - based on the desired product - easily be chosen by a skilled person in this field and will be illustrated in the examples, without being limited hereto.

Preferred UVA-filter substances i.e. substances having absorption maximums between about 340 nm and 400 nm suitable for the incorporation into topical compositions according to the invention may be organic or inorganic compounds such as e.g. Phenylene-1,4-bis-benzimidazolsulfonic acids or salts such as 2,2-(1,4-phenylene)bis-(1H-benzimidazol-4,6-disulfonic acid) (Neoheliopan AP); Amino substituted hydroxybenzophenones such as 2-(4-Diethylamino-2-hydroxy-benzoyl)-benzoic acid hexylester (Uvinul A plus) as described in the European Patent Publication EP 1046391; Pigments such as microparticulated ZnO and the like. The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art.

Preferred topical compositions according to the invention further comprise one or several antioxidant(s). Suitable antioxidants for the incorporation into the topical compositions according to the invention are all antioxidant suitable for cosmetic applications. Particular preferred are water soluble antioxidants such as vitamins such as e.g. ascorbic acid as well as derivatives thereof such as e.g. ascorbyl phosphate such as Stay C (sodium ascorbyl monophosphate) from DSM Nutritional Products Ltd.

Further preferred antioxidants are BHT, vitamin E and derivatives thereof as well as vitamin A and derivatives thereof.

The amount of antioxidant (one or several) in the topical compositions according to the invention is preferably selected in the range of about 0.001 to 30 wt.-%, in particular in the range of about 0.05 to 20 wt.-%, most particular in the range of about 0.1 to 10 wt.-%, with respect to the total amount of the topical composition.

If a vitamin E derivative is used in the topical compositions according to the invention preferably tocopheryl acetate is used. Tocopheryl acetate may be present in the topical preparations in an amount from about 0.05 to 25 wt.-%, in particular 0.5 to 5 wt.-%. Another vitamin E derivative of interest is tocopheryl linoleate. Tocopheryl linoleate may be present in the skin care composition in an amount from about 0.05 to 25 wt.-% in particular 0.5 to 5 wt.-%.

Vitamin A and/or its derivatives in particular retinoid derivatives such as retinyl palmitate or retinyl propionate is preferably used in the topical preparations according to the invention in an amount of 0.01 to 5 wt.-%, in particular 0.01 to 0.3 wt.-%.

Preferably, the topical compositions according to the invention further comprise a fatty alcohol, such as in particular cetyl alcohol, cetearyl alcohol and/ or behenyl alcohol. The total amount of one or several fatty alcohols on the topical compositions according to the invention is preferably selected in the range of about 0.1 to 10.0 wt.-%, in particular in the range of about 0.5 to 6.0 wt.-% with respect to the total weight of the topical composition.

Suitable cosmetically active ingredients according to the invention encompass agents suitable for skin lightening; tanning prevention; self tanning; treatment of hyperpigmentation; preventing or reducing acne; treatment of wrinkles, lines, atrophy and/or inflammation; treatment of cellulites (e.g. phytanic acid), firming, energizing, skin soothing, as well as agents to improve skin elasticity and skin barrier.
The cosmetically active ingredients useful herein can in some instances provide more than one benefit or operate via more than one mode of action.

Preferred examples of cosmetically active ingredients for the incorporation into topical compositions according to the invention encompass vitamin B₆, vitamin B₁₂, biotin, co-enzyme Q10, EGCG, alpha-liponic acid, phytoen, hydroxytyrosol and/or olive extract, shea butter, algae extract, cocoa butter, aloe extract, jojoba oil, echinacea extract, chamomile extract, alpha-glucosylrutin, carnitin, carnosine, natural and/ or synthetic isoflavanoids, creatin, taurin, alanine, glycyrrhetinic acid, glycyryca glabra and/ or glycyrrhiza inflata.

The cosmetically active ingredient is typically included in an amount of at least 0.001 wt. % based on the total weight of the topical preparation. Generally, an amount of about 0.001 wt. % to about 30 wt. %, preferably from about 0.001 wt. % to about 10 wt. % of an additional cosmetically active agent is used.

Further preferred the topical compositions according to the invention further comprise a moisturizer. Moisturizers are chemical agents specially designed to make the external layers of the skin (epidermis) softer and more pliable by increasing its hydration (water content) which can be determined e.g. by measuring the transepidermal water loss (TEWL). Suitable moisturizer according to the invention are for example glycerin, lactic acid and/ or lactate such as in particular sodium lactate, butyleneglycol, propyleneglycol, biosaccaride gum-1, glycine soja, ethylhexyloxyglycerine, pyrrolidoncarbonic acid and/or urea.

Suitable preservatives according to the invention encompass for example formaldehyde releasing chemicals such as e.g. DMDM Hydantoin (e.g. as Glydant by Lonza), iodopropylbutylcarbamate (e.g. as Glycacil-L, Glycacil-S by Lonza and/ or Dekaben LMB by Jan Dekker), parabens (e.g. p-hydroxybenzoic acid alkyl ester such as methyl-, ethyl-, propyl- and/ or butylparaben), phenoxyethanol, ethanol, benzoic acid without being limited thereto.

Preferred complexing agents include EDTA, [S,S]-Ethylendiamindisuccinat (EDDS) (e.g. as Octaquest by. Octel), Pentasodium-Ethylendiaminetetramethylenphosphonate (e.g. as Dequest 2046 by Monsanto) and/ or iminodisuccinates (e.g. as Iminodisuccinat VP OC 370 or Baypure CX 100 by Bayer).

Preferably, the topical compositions according to the invention comprise a thickener in particular if the topical composition is in the form of an emulsion to assist in making the consistency of a product suitable. Preferred thickeners are aluminiumsilicates, xanthan gum, hydroxypropylmethylcellulose, polyacrylates such as carbopole^{®} (e.g. Carbopole 980, 981, 1382, 2984, 5984) or mixtures thereof. Further preferred thickeners encompass acrylate/C₁₀₋₃₀ alkyl acrylate copolymers (such as e.g. Pemulen TR 1, Pemulen TR 2, Carbopol 1328 by. NOVEON) as well as Aristoflex AVC (INCI: Ammonium Acryloyldimethyltaurate/VP Copolymer).

Of course, one skilled in this art will take care to select the above mentioned optional additional compound or compounds and/or their amounts such that the advantageous properties intrinsically associated with the combination in accordance with the invention are not, or not substantially, detrimentally affected by the envisaged addition or additions.

The cosmetic and/ or dermatological compositions according to the invention have a pH in the range of 3-10, preferably in the range of pH of 4-8, most preferred in the range of pH 6-8.

The following examples are provided to further illustrate the compositions and effects of the present invention. These examples are illustrative only and are not intended to limit the scope of the invention in any way.

### Example 1

Various O/W emulsions according to table 1 were prepared and tested. The results are also presented in table 1.

**Table 1:**

| | **Nr.** | **1** | **2** | **3** | **4** | **5** | **6** |
|---|---|---|---|---|---|---|---|
| | INCl | wt.-% | | | | | |
| **A** | Butyl Methoxy-dibenzoylmethane | 5 | 5 | 5 | 5 | 5 | 5 |
| | Polysilicone-15 | 3 | 3 | 3 | 3 | 3 | 3 |
| | Octocrylene | 8 | 8 | 4.5 | 4.5 | 4.5 | 4.5 |
| | Titanium Dioxide | 6 | 6 | 3 | 3 | | |
| | Ethylhexyl Salicylate | | | 5 | 5 | 5 | 5 |
| | Homosalate | | | | | 7.5 | 7.5 |
| | Potassium Cetyl Phosphate | | 2 | | 2.5 | | 2 |
| | Glyceryl Stearate & PEG-100 Stearate | 3.5 | | 3.5 | | 3.5 | |
| | Tocopheryl Acetate | 0.5 | 0.5 | 0.5 | 0.5 | 0.5 | 0.5 |
| | Dimethicone 200/100 cs or Dimethicone 200/350 cs | 0.3 | 0.3 | 1 | 0.3 | 0.3 | 0.3 |
| | BHT | 0.05 | | | | | |
| | Behenyl Alcohol, Cetyl Alcohol or Cetearyl Alcohol | 1.5 | 2 | 2 | 2 | 2 | 2 |
| | Phenoxyethanol & Methylparaben & Ethylparaben & Butylparaben & Propylparaben & Isobutylparaben | 0.8 | 0.8 | 0.8 | 0.8 | 0.8 | 0.8 |
| | Cyclopentasiloxane & Cyclohexasiloxane | 1.5 | 1.5 | 2 | 1.5 | 1.5 | 1.5 |
| | Diisopropyl Sebacate | 12 | 12 | 10 | 12 | 10 | 12 |
| | C12-15 Alkyl Benzoate | 12 | 10 | 10 | 10 | 10 | 10 |
| | Butylene Glycol Dicaprylate/Dicaprate | | | 5 | | 5 | |
| | VP/Eicosene Copolymer | | 2 | | 2 | | 2 |
| **B** | Triethanolamine | q.s. | | | | | |
| | Glycerin | 3 | 3 | 3 | 3 | 3 | 3 |
| | Xanthan Gum | 0.2 | 0.3 | 0.2 | 0.2 | 0.2 | 0.3 |
| | Disodium EDTA or Pentasodium Ethylenediamine Tetramethylene Phosphonate | 0.1 | 0.5 | 0.1 | 0.5 | 0.1 | 0.5 |
| | Aqua | Ad 100 | | | | | |
| **C** | Phenylbenzimidazole Sulfonic Acid | 4 | 4 | 4 | 4 | 4 | 4 |
| | Aqua | 10 | 10 | 10 | 10 | 10 | 10 |
| | Triethanolamine (TEA) | q.s. | | | | | |
| | in vivo SPF | 46 | 53 | 41 | 76 | 45 | 49 |
| | UVA/UVB = 1/3* | yes | yes | yes | yes | yes | yes |
| | Critical wavelengt | 377 | 377 | 377 | 376 | 376 | 376 |
| | Photostable | Yes | Yes | Yes | Yes | Yes | Yes |
| | **Total amount of UV-filter substances** | **26** | **26** | **24.5** | **24.5** | **29** | **29** |

| | | | | | | | |
|---|---|---|---|---|---|---|---|
| *EU recommendation fulfilled, determined according to the 'Method for the in vitro determination of UVA protection provided by sunscreen products' (COLIPA Guideline 2007). | | | | | | | |

Procedure for preparation of the formulations: Heat part A to 85°C and stir until homogeneous then heat part B to 75°C and add to part A under agitation, then cool down to 55 °C and add TEA. When everything is homogenous add part C pre-heated to 50°C. Be sure that the pH of Phenylbenzimidazole Sulfonic Acid solution is 7.0 (adjusted with TEA). If traces remain, add small quantities of the used neutralizing base until the particles are dissolved. Homogenize thoroughly and then with continued mixing cool down to ambient temperature. It is generally recommended to use vacuum while producing the emulsion.

As can be seen from table 1 stable, high in vivo SPF compositions can be obtained meeting the Colipa UVA recommendations as well as the critical wavelength always using a combination of polysilicones-15, butyl methoxydibenzoylmethane and phenylbenzimidazole sulfonic acid in combination with further UVB and/ or broadband filters at relatively low total UV-filter concentrations.

## Claims

1. Topical composition comprising at least 3 wt.-% of polysilicone-15, at least 3 wt.% of phenylbenzimidazol sulfonic acid, at least 4.5 wt.-% of butyl methoxydibenzoylmethane and at least 10 wt.-% of at least one further UVB and/ or broadband-filter substance in a cosmetically acceptable carrier, **characterized in that** the at least one further UVB and/ or broadband-filter substance is selected from the group consisting of octocrylene, ethyl hexylsalicylate,homosalate, unsymmetrical s-triazine derivatives, 2-Hydroxy-4-methoxy-benzophenon, 2,2'-Methylen-bis-(6-(2H-benzotriazol-2-yl)-4-(1,1,3,3-tetramethyl-butyl)-phenol), and titanium dioxide.

2. Topical composition according to claim 1 comprising at least 12 wt.-% of the at least one further UVB and/ or broadband-filter substance.

3. Topical composition according to claim 1 or 2, wherein at least two further UVB and/ or broadband-filter substances are present.

4. Topical composition according to any one of claims 1 to 3, wherein one of the UVB and/ or broadband-filter substances is octocrylene.

5. Topical composition according to claim 4, wherein a further UVB and/ or broadband-filter substance selected from titanium dioxide, ethylhexyl salicylate and/ or homosalate is present.

6. Topical composition according to any one of claims 1 to 5, which is an O/W emulsion.

7. Topical composition according to any one of claims 1 to 6, wherein a phosphate ester surfactant is present.

8. Topical composition according to claim 7, wherein the phosphate ester surfactant is selected from cetyl phosphate, potassium cetyl phosphate, and/ or DEA cetyl phosphate.

9. Topical composition according to any one of claims 1 to 8, wherein the total amount of UV-filter substances is in the range of 20 to 35 wt.-% with respect to the total weigh of the topical composition and the ratio of the in vivo SPF to the UV-filter concentration is in the range of about 2 to 3.

## Patentansprüche

1. Topische Zusammensetzung, umfassend mindestens 3 Gew.-% Polysilicon-15, mindestens 3 Gew.-% Phenylbenzimidazolsulfonsäure, mindestens 4,5 Gew.-% Butylmethoxydibenzoylmethan und mindestens 10 Gew.-% mindestens einer weiteren UVB- und/oder Breitbandfiltersubstanz in einem kosmetisch unbedenklichen Träger, **dadurch gekennzeichnet, dass** die mindestens eine weitere UVB- und/oder Breitbandfiltersubstanz ausgewählt ist aus der Gruppe bestehend aus Octocrylen, Salicylsäureethylhexylester, Homosalat, unsymmetrischen s-Triazinderivaten, 2-Hydroxy-4-methoxybenzophenon, 2,2'-Methylen-bis-(6-2H-benzotriazol-2-yl)-4-(1,1,3,3-tetramethylbutyl)phenol) und Titandioxid.

2. Topische Zusammensetzung nach Anspruch 1, umfassend mindestens 12 Gew.-% der mindestens einen weiteren UVB- und/oder Breitbandfiltersubstanz.

3. Topische Zusammensetzung nach Anspruch 1 oder 2, wobei mindestens zwei weitere UVB- und/oder Breitbandfiltersubstanzen vorhanden sind.

4. Topische Zusammensetzung nach einem der Ansprüche 1 bis 3, wobei es sich bei einer der UVB- und/oder Breitbandfiltersubstanzen um Octocrylen handelt.

5. Topische Zusammensetzung nach Anspruch 4, wobei eine weitere, aus Titandioxid, Salicylsäureethylhexylester und/oder Homosalat ausgewählte UVB- und/oder Breitbandfiltersubstanz vorhanden ist.

6. Topische Zusammensetzung nach einem der Ansprüche 1 bis 5, bei der es sich um eine O/W-Emulsion handelt.

7. Topische Zusammensetzung nach einem der Ansprüche 1 bis 6, wobei ein Phosphatestertensid vorhanden ist.

8. Topische Zusammensetzung nach Anspruch 7, wobei das Phosphatestertensid ausgewählt ist aus Cetylphosphat, Kaliumcetylphosphat und/oder DEA-Cetylphosphat.

9. Topische Zusammensetzung nach einem der Ansprüche 1 bis 8, wobei die Gesamtmenge an UV-Filtersubstanzen, bezogen auf das Gesamtgewicht der topischen Zusammensetzung, im Bereich von 20 bis 35 Gew.-% liegt und das Verhältnis der invivo-SPF zur UV-Filter-Konzentration im Bereich von etwa 2 bis 3 liegt.

## Revendications

1. Composition topique comprenant au moins 3% en poids de polysilicone-15, au moins 3% en poids d'acide phénylbenzimidazolsulfonique, au moins 4,5% en poids de méthoxydibenzoylméthane de butyle et au moins 10% en poids d'au moins une autre substance de filtre UVB et/ou à large bande dans un véhicule cosmétiquement acceptable, **caractérisée en ce que** la au moins une autre substance de filtre UVB et/ou à large bande est choisie dans le groupe constitué par l'octocrylène, le salicylate d'éthylhexyle, l'homosalate, les dérivés de s-triazine non symétriques, la 2-hydroxy-4-méthoxybenzophénone, le 2,2'-méthylène-bis-(6-(2H-benzotriazol-2-yl)-4-(1,1,3,3-tétraméthylbutyl)phénol) et le dioxyde de titane.

2. Composition topique selon la revendication 1, comprenant au moins 12% en poids de la au moins une autre substance de filtre UVB et/ou à large bande.

3. Composition topique selon la revendication 1 ou 2, dans laquelle au moins deux autres substances de filtre UVB et/ou à large bande sont présentes.

4. Composition topique selon l'une quelconque des revendications 1 à 3, dans laquelle l'une des substances de filtre UVB et/ou à large bande est l'octocrylène.

5. Composition topique selon la revendication 4, dans laquelle une autre substance de filtre UVB et/ou à large bande choisie parmi le dioxyde de titane, le salicylate d'éthylhexyle et/ou l'homosalate, est présente.

6. Composition topique selon l'une quelconque des revendications 1 à 5, qui est une émulsion H/E.

7. Composition topique selon l'une quelconque des revendications 1 à 6, dans laquelle un agent tensioactif à base d'ester de phosphate est présent.

8. Composition topique selon la revendication 7, dans laquelle l'agent tensioactif à base d'ester de phosphate est choisi parmi le phosphate de cétyle, le phosphate de cétyle potassique et/ou le phosphate de cétyle-DEA.

9. Composition topique selon l'une quelconque des revendications 1 à 8, dans laquelle la quantité totale de substances de filtre UV se trouve dans la plage allant de 20 à 35% en poids par rapport au poids total de la composition topique et le rapport du FPS *in vivo* à la concentration en filtre UV se trouve dans la plage allant d'environ 2 à 3.
